Europäisches Patentamt

European Patent Office

Office européen des brevets

(11) Veröffentlichungsnummer: **0 424 947 A1**

# (12) EUROPÄISCHE PATENTANMELDUNG

(21) Anmeldenummer: 90120514.6

(22) Anmeldetag: **26.10.90**

(51) Int. Cl.⁵: **A61B 3/12**

(30) Priorität: **27.10.89 DE 8912757 U**

(43) Veröffentlichungstag der Anmeldung:
**02.05.91 Patentblatt 91/18**

(84) Benannte Vertragsstaaten:
**AT CH DE ES FR GB IT LI NL SE**

(71) Anmelder: **Firma Carl Zeiss**

**W-7920 Heidenheim (Brenz)(DE)**

(84) **CH DE ES FR IT LI NL SE AT**

(71) Anmelder: **CARL ZEISS-STIFTUNG HANDELND ALS CARL ZEISS**

**W-7920 Heidenheim (Brenz)(DE)**

(84) **GB**

(72) Erfinder: **Blaha, Erich**
**Staufenstrasse 15**
**W-7087 Essingen(DE)**
Erfinder: **Gaida, Gerhard, Dr.**
**Hüttfeldstrasse 14**
**W-7080 Aalen(DE)**

(54) **Konfokales Rasterophthalmoskop.**

(57) Ein konfokales Rasterophthalmoskop, besteht aus einem auf das Patientenauge ausrichtbaren Geräteoberteil (26) und aus einem Versorgungsblock (27). Der Versorgungsblock (27) enthält einen oder mehrere Laser (9) zur Erzeugung des Meßlichts und einen Sekundärelektronenvervielfacher (25) zur Detektion des am Augenhintergrund des Patienten zurückgestreuten Lichts.

Zwischen dem Versorgungsblock (27) und dem Geräteoberteil (26) sind zwei flexible Lichtwellenleiter (7, 8) vorgesehen; ein erster (7), der das Licht des Lasers (9) zum Geräteoberteil (26) leitet, und ein zweiter (8), der das am Augenhintergrund zurückgestreute Licht zum Sekundärelektronenvervielfacher (25) leitet.

Durch die räumliche Trennung der Laser (9) und des Sekundärelektronenvervielfachers (25) vom Geräteoberteil (26) ist dieses besonders kompakt und daher leicht auf das Patientenauge ausrichtbar. Außerdem wird die Einkopplung elektromagnetischer Streufeldern, die am Galvanoscanner (18) austreten, in die dem Sekundärelektronenvervielfacher (25) nachgeschalteten Datenleitungen vermieden.

Fig.2

## KONFOKALES RASTEROPHTHALMOSKOP

Die vorliegende Erfindung betrifft ein konfokales Rasterophthalmoskop mit einer einzigen, in einem gemeinsamen Teil des Beleuchtungs- und Beobachtungsstrahlenganges angeordneten Lichtablenkeinrichtung zur punktweisen Abtastung des Augenhintergrundes mit dem Licht einer Punktlichtquelle, wobei ein flexibler Lichtwellenleiter vorgesehen ist, der das von einer realen Lichtquelle ausgesandte Licht der Punktlichtquelle zuführt, mit einer Lochblende, die in einer zur Punktlichtquelle konjugierten Ebene im Beobachtungsstrahlengang angeordnet ist, und mit einem Detektor zur Messung des durch die Lochblende transmittierten Lichts.

Ein solches konfokales Rasterophthalmoskop ist beispielsweise in der EP-OS 0 145 563 beschrieben. Es dient zur Untersuchung des Augenhintergrundes mit hoher Auflösung. Dazu wird der Lichtstrahl einer Punktlichtquelle mit Hilfe einer Lichtablenkeinrichtung rasterförmig abgelenkt und auf den Augenhintergrund abgebildet. Das am Augenhintergrund reflektierte Licht durchleuchtet dieselbe Lichtablenkeinrichtung in entgegengesetzter Richtung und wird anschließend von einem Strahlteiler in den Beobachtungsstrahlengang gelenkt. Zur Verbesserung des Kontrastes und der Auflösung ist im Beobachtungsstrahlengang in einer zur Ebene der Punktlichtquelle konjugierten Ebene eine Lochblende vorgesehen. Ein Detektor detektiert das durch die Lochblende transmittierte Licht. Der Durchmesser der Lochblende ist so gewählt, daß nur das am Augenhintergrund reflektierte Licht in den Detektor gelangt. Das Ausgangssignal des Detektors wird einem Bildspeicher und einem Monitor zugeführt, der mit der Lichtablenkeinrichtung synchronisiert ist und der damit ein TV-Bild des beleuchteten Augenbereiches darstellt.

Die Punktlichtquelle kann durch die Strahltaille eines fokussierten Laserstrahls realisiert sein. Zur physischen Trennung zwischen dem Laser und dem Teil des Ophthalmoskops, der sich nahe beim Patienten befindet, kann zwischen dem Laser und dem die Strahltaille erzeugenden Objektiv ein Lichtwellenleiter angeordnet sein. Es ist dann eine kompaktere Bauweise des auf das Patientenauge auszurichtenden Geräteteils möglich.

Bei diesem Ophthalmoskop erzeugt die Lichtablenkeinrichtung, die aus einem Spiegelrad- und einem Galvanoscanner besteht, elektromagnetische Streufelder, die aufgrund der räumlichen Nähe zwischen der Ablenkreinrichtung und dem Detektor sehr leicht auf der Ausgangsseite des Detektors und in der darauffolgenden Signalverarbeitung zu Störsignalen führen. Zur Vermeidung solcher Störsignale sind aufwendige Abschirmmaßnahmen notwendig.

Bei besonders lichtempfindlichen Ophthalmoskopen ist der Detektor ein Sekundärelektronenvervielfacher. Da der Sekundärelektronenvervielfacher mit einer Hochspannung von ca. 1000 V betrieben wird, sind aus Grunden der Gerätesicherheit sehr kostenintensive Schutzvorkehrungen zu treffen. Diese Schutzvorkehrungen beanspruchen außerdem sehr viel Platz.

Eine sehr kompakte Bauweise eines solchen Ophthalmoskops ist aber andererseits wünschenswert, da zur Untersuchung der Untersuchende das Ophthalmoskop auf das Patientenauge ausrichten muß, indem er seitlich an dem Ophthalmoskop vorbei auf das Patientenauge sieht und den dort vom Ophthalmoskop erzeugten Lichtfleck beobachtet. Eine kompakte Bauweise des Ophthalmoskops ermöglicht daher eine bequeme und schnelle Ausrichtung des Ophthalmoskops auf das Auge des Patienten.

Es ist die Aufgabe der vorliegenden Erfindung, ein Ophthalmoskop der eingangs genannten Art zu schaffen, dessen auf den Patienten auszurichtendes Teil kompakter ist als bei dem vorbekannten Gerät, und bei dem das Auftreten von Störsignalen als Folge der von der Lichtablenkeinrichtung erzeugten und in die elektronischen Datenleitungen einsprechenden Streufelder vermieden ist.

Diese Aufgabe wird der Erfindung gemäß dadurch gelöst, daß zwischen der Lochblende und dem Detektor ein zweiter, flexibler und mehrmodiger Lichtwellenleiter vorgesehen ist, und daß der Detektor in einem separaten Geräteteil angeordnet ist, das relativ zu dem die Lichtablenkeinrichtung enthaltenden Geräteteil beweglich ist.

Der zweite Lichtwellenleiter ermöglicht eine Führung des Lichts über eine größere Strecke, die möglicherweise bis zu einigen Metern betragen kann. Die Streufelder der Lichtablenkeinrichtung sind in dieser Entfernung kaum noch beobachtbar. Da der Lichtwellenleiter flexibel ist, braucht der Detektor bei der Ausrichtung des Ophthalmoskops auf das Auge des Patienten nicht mit ausgerichtet zu werden. Er ist stattdessen in einem separaten Gehäuse angeordnet, das während der Untersuchung ortsfest ist. Der auf das Auge des Patienten auszurichtende Teil des Ophthalmoskops kann dann entsprechend kompakter gebaut sein.

Wenn der Detektor ein Sekundärelektronenvervielfacher ist, so wird durch die räumliche Trennung zwischen dem Sekundärelektronenvervielfacher und dem auf das Patientenauge auszurichtenden Geräteteil eine Gefährdung des Patienten oder des Untersuchenden ausgeschlossen. Besonders teure und Raum beanspruchende Maßnahmen sind dafür nicht erforderlich.

Der Einsatz von Glasfasern zur Bildübertragung ist zwar bei ophthalmischen Geräten aus der US-PS 36 98 099 und der US-PS 47 11 542 bekannt. Die Glasfasern dienen hier jedoch lediglich zum Transport des Bildes oder des zurückgestreuten Lichts auf einem flexiblen optischen Weg. Dagegen ist in diesen Patentschriften nicht vorgesehen, daß von einer Lichtablenkeinrichtung ausgehende und in die Datenleitungen einstreuende Störsignale vermieden werden. Außerdem handelt es sich bei den beschriebenen ophthalmischen Geräten auch nicht um Rasterophthalmoskope.

Vorteilhaft ist es, wenn die Lichtquelle und der Detektor eine räumliche Einheit bilden, in der auch noch die elektronische Signalverarbeitung untergebracht sein kann. Das Ophthalmoskop besteht dann aus einem ersten, auf das Auge des Patienten auszurichtenden und einem zweiten, ortsfesten Geräteteil.

Der Lichtwellenleiter zwischen der Lochblende und dem Detektor ist vorteilhafterweise mehrmodig. Ein solcher Multimodewellenleiter ist dicker und daher nicht so sehr justierempfindlich wie ein Monomodewellenleiter. Damit ein möglichst großer Anteil des durch die Lochblende transmittierten Lichts in den Lichtwellenleiter einkoppelbar ist, sollte dessen Kerndurchmesser größer als der Durchmesser der Lochblende sein.

Der Kern des Lichtwellenleiters kann jedoch auch selbst die Lochblende darstellen. In diesem Fall sollte der Kerndurchmesser des Lichtwellenleiters dem Durchmesser der somit eingesparten Lochblende entsprechen.

Im folgenden wird ein Ausführungsbeispiel der vorliegenden Erfindung anhand zweier Figuren näher beschrieben. Im einzelnen zeigen:

Figur 1 eine perspektivische Darstellung eines konfokalen Rasterophthalmoskops gemäß der Erfindung;

Figur 2 eine schematische Darstellung des Strahlenganges in einem konfokalen Rasterophthalmoskop gemäß der Erfindung;

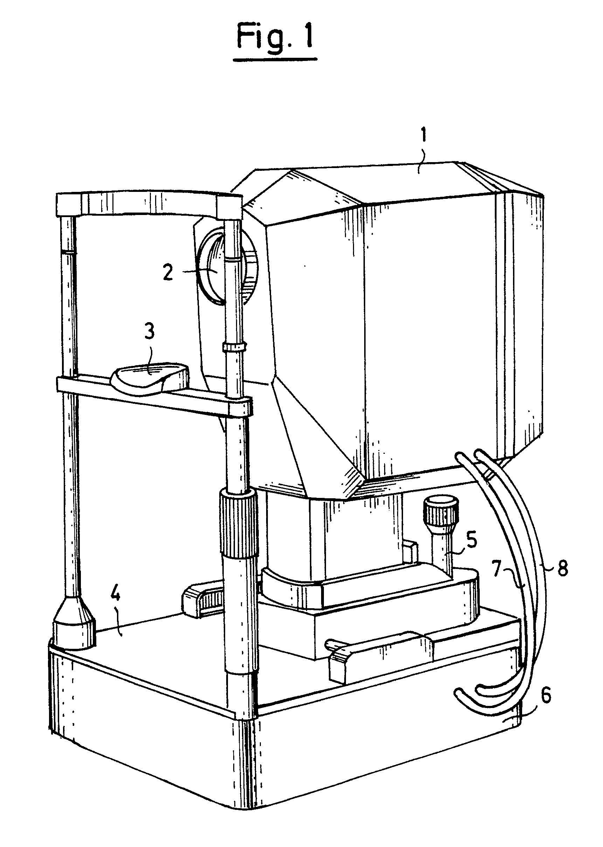

In Fig. 1 ist mit (1) das Oberteil eines konfokalen Rasterophthalmoskops bezeichnet. Aus einer Öffnung (2) tritt ein Laserstrahl aus diesem Oberteil aus. Der Laserstrahl wird in das Auge eines Patienten fokussiert. Zur Stabilisierung des Patientenkopfes ist eine Kinnstütze (3) vorgesehen, die auf einer Grundplatte (4) höhenverstellbar angeordnet ist. Zur Ausrichtung und zur Fokussierung des Laserstrahls ist das Oberteil (1) mit Hilfe des Stellhebels (5) parallel zur Grundplatte (4) verschiebbar. Unter der Grundplatte (4) ist ein Versorgungsblock (6) angeordnet, der einen Laser, einen Sekundärelektronenvervielfacher und auch eine elektronische Signalverarbeitung enthält. Ein erster, flexibler und einmodiger Lichtwellenleiter (7) leitet das vom Laser ausgesandte Licht zum Geräteoberteil (1). Ein zweiter, mehrmodiger und ebenfalls flexibler Lichtwellenleiter (8) führt das Licht des Beobachtungsstrahlenganges dem Sekundärelektronenvervielfacher zu.

Für Farbdarstellungen kann der Versorgungsblock (6) auch mehrere Laser unterschiedlicher Wellenlänge enthalten, deren Lichtstrahlen in den ersten Lichtwellenleiter (7) eingekoppelt werden

Der Versorgungsblock (6) muß nicht unter der Grundplatte (4) angeordnet sein. Er kann auch einige Meter von dem Geräteoberteil (1) entfernt sein.

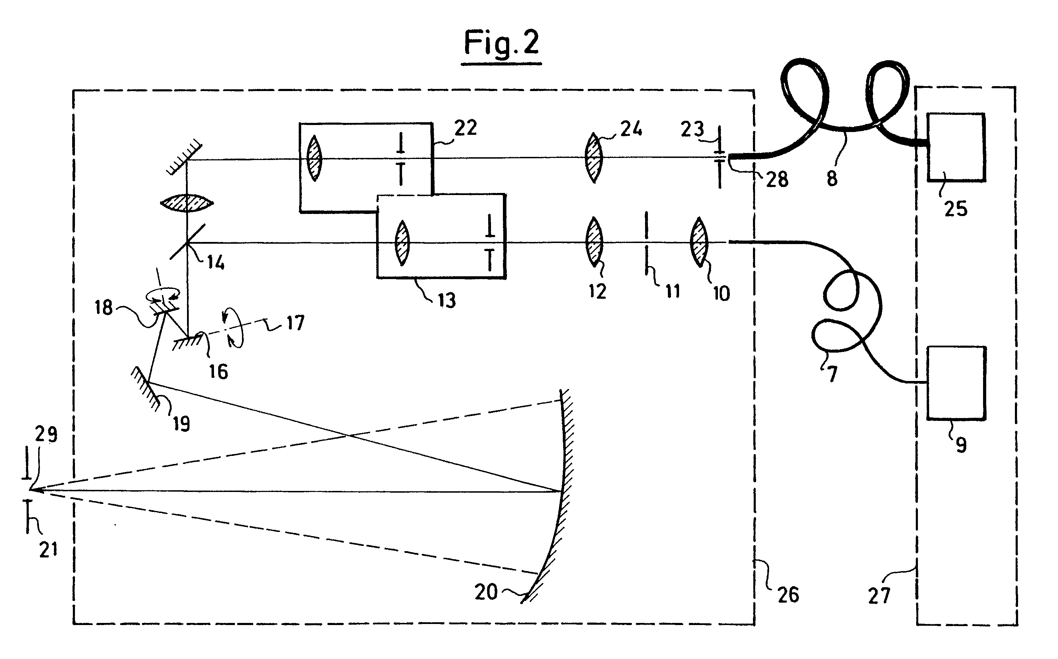

In der schematischen Darstellung von Figur 2 ist nur ein einziger Laser (9) dargestellt. Das Licht dieses Lasers (9) wird über einen Lichtwellenleiter (7) dem Objektiv (10) zugeführt. Der Lichtwellenleiter (7) ist ein Monomodelichtwellenleiter mit einem Kerndurchmesser von ca. 6 $\mu$m. Das dem Objektiv (10) zugeführte Licht wird von diesem Objektiv (10) in die Ebene (11) fokussiert. Der in der Ebene (11) liegende Laserfokus stellt eine Punktlichtquelle dar.

Ein zweites Objektiv (12) sammelt das Licht dieser Punktlichtquelle auf und führt es über eine zur Kompensation einer Fehlsichtigkeit des Patienten dienende Einrichtung (13) und einen Strahlteiler (14) einer Lichtablenkeinrichtung zu. Die Lichtablenkeinrichtung besteht aus einem um die Achse (17) rotierenden Spiegelradscanner (16) und einem Galvanoscanner (18). Während der Spiegelradscanner (16) das Licht senkrecht zur Zeichenebene ablenkt, lenkt es der Galvanoscanner (18) in der Zeichenebene (18) ab, so daß der Laserstrahl die Ebene die senkrecht zu seiner Ausbreitungsrichtung liegt, abrastert.

Hinter dieser Lichtablenkeinrichtung wird das Licht über einen Umlenkspiegel (19) und einen Hohlspiegel (20) auf den Augenhintergrund des Patienten fokussiert. Der Augenhintergrund ist hier als Blende (21) dargestellt. Die Ablenkung des Lichtstrahls durch den Spiegelradscanner (16) und den Galvanoscanner (18) bewirkt eine punktweise Abtastung des Augenhintergrundes.

Das von dem Augenhintergrund reflektierte Licht wird von dem Hohlspiegel (20) wieder aufgesammelt und durchläuft über den Umlenkspiegel (19) die Lichtablenkeinrichtung in entgegengesetzter Richtung. Der Strahlteiler (14) lenkt dann dieses zurückreflektierte Licht aus dem gemeinsamen Teil des Beleuchtungs- und Beobachtungsstrahlenganges hinaus. Nach dem Durchgang durch eine weitere Einrichtung (22), die der Einrichtung zur Kompensation der Fehlsichtigkeit des Patienten entspricht und mit dieser gekoppelt ist, erzeugt ein drittes Objektiv (24) in der Ebene einer Lochblende (23) ein Bild des von dem Hohlspiegel (20) erzeugten Laserfokus (29).

Der Durchmesser der Lochblende (23) ist gerade so gewählt, daß nur das Licht des Laserfokus (29) und damit das am Augenhintergrund reflektierte Licht durch die Lochblende (23) transmittiert wird. Das vor oder hinter dem Laserfokus (29) reflektierte oder gestreute Licht wird dagegen von der Lochblende (23) absorbiert. Durch die Lochblende (23) wird somit eine Verbesserung des Kontrastes und der Auflösung erzielt.

Entscheidend an der vorliegenden Erfindung ist, daß das durch die Lochblende (23) transmittierte Licht in einen flexiblen Multimodelichtwellenleiter (8) eingekoppelt wird. Da der Multimodelichtwellenleiter (8) einen Kerndurchmesser von ca. 0,3 mm hat, ist seine Justierung im Bereich der Lochblende (23) relativ unkritisch. Bewegungen des Multimodelichtwellenleiers (8) können Transmissionsverluste in diesem Lichtwellenleiter (8) verursachen. Zur Vermeidung dieser Transmissionsverluste ist das Objektiv (24) so gewählt, daß die numerische Apertur des Multimodelichtwellenleiters einen Wert zwischen 0,1 und 0,5 hat.

Der Multimodewellenleiter (8) führt das durch die Lochblende (23) transmittierte Licht einem Sekundärelektronenvervielfacher (25) zu. Das Ausgangssignal des Sekundärelektronenvervielfachers (25) ist einem nicht dargestellten Bildspeicher und einem ebenfalls nicht dargestellten Monitor zugeführt. Der Monitor ist mit der aus dem Spiegelradscanner (16) und dem Galvanoscanner (18) bestehenden Lichtablenkeinrichtung synchronisiert. Auf dem Monitor wird somit ein Bild des Augenhintergrundes dargestellt.

Durch die Verwendung zweier Lichtwellenleiter (7, 8) sind sowohl der Laser (9) als auch der Sekundärelektronenvervielfacher (25) räumlich von dem gestrichelt dargestellten Geräteoberteil (26) getrennt. Diese Trennung kann je nach der Länge der beiden Lichtwellenleiter (7, 8) bis zu einige Meter betragen. Ein Einsprechen der aus der Lichtablenkeinrichtung austretenden elektromagnetischen Streufelder in das Ausgangssignal des Sekundärelektronenvervielfachers (25) ist durch diese räumliche Trennung vermieden.

Die Flexibilität der beiden Lichtwellenleiter (7, 8) ermöglicht eine Ausrichtung des Geräteoberteils (26) und damit des Laserfokus (24) auf den Augenhintergrund des Patienten, während sowohl der Laser (9) als auch der Sekundärelektronenvervielfacher (25) ortsfest sind. Der Sekundärelektronenvervielfacher (25) und der Laser (9) sind in einem gemeinsamen, gestrichelt dargestellten Geräteblock (27) angeordnet.

Da das auf das Patientenauge auszurichtende Geräteoberteil (26) weder den Laser (9) noch einen Sekundärelektronenvervielfacher (25) enthält, ist eine sehr kompakte Bauweise dieses Geräteoberteils möglich. Diese kompakte Bauweise ermöglicht dem Untersuchenden einen freien Blick auf das Patientenauge, wodurch eine schnelle und sichere Ausrichtung des Geräteoberteils (26) möglich ist.

Wenn der Kerndurchmesser des Multimodelichtwellenleiters (8) dem Durchmesser der Lochblende (23) entspricht, kann der Kern des Multimodelichtwellenleiters (8) die Lochblende (23) ersetzen. In diesem Fall ist die Stirnflache (28) des Lichtwellenleiters (8) exakt in der Ebene der Lochblende (23) zu positionieren.

## Ansprüche

1. Konfokales Rasterophthalmoskop mit einer einzigen, in einem gemeinsamen Teil des Beleuchtungs- und Beobachtungsstrahlengangs angeordneten Lichtablenkeinrichtung zur punktweisen Abtastung des Augenhintergrundes mit dem Licht einer Punktlichtquelle, wobei ein flexibler Lichtwellenleiter vorgesehen ist, der das von einer realen Lichtquelle ausgesandte Licht der Punktlichtquelle zuführt, mit einer Lochblende, die in einer zur Punktlichtquelle konjugierten Ebene im Beobachtungsstrahlengang angeordnet ist, und mit einem Detektor zur Messung des durch die Lochblende transmittierten Lichts, dadurch gekennzeichnet, daß zwischen der Lochblende (23) und dem Detektor (25) ein zweiter, flexibler und mehrmodiger Lichtwellenleiter (8) vorgesehen ist, und daß der Detektor (25) in einem separaten Geräteteil angeordnet ist, das relativ zu dem die Lichtablenkeinrichtung enthaltenden Geräteteil (26) beweglich ist.

2. Konfokales Rasterophthalmoskop nach Anspruch 1, dadurch gekennzeichnet, daß die reale Lichtquelle (9) und der Detektor (25) in einem gemeinsamen Geräteteil (27) angeordnet sind.

3. Konfokales Rasterophthalmoskop nach Anspruch 2, dadurch gekennzeichnet, daß als Detektor ein Sekundärelektronenvervielfacher vorgesehen ist.

4. Konfokales Rasterophthalmoskop nach einem der Ansprüche 1 bis 3, dadurch gekennzeichnet, daß der Kerndurchmesser des zwischen der Lochblende (23) und dem Detektor (25) angeordneten Lichtwellenleiters (8) großer als der Durchmesser der Lochblende (23) ist.

5. Konfokales Rasterophthalmoskop nach einem der Ansprüche 1 bis 3, dadurch gekennzeichnet, daß der Kern des zwischen der Lochblende (23) und dem Detektor (25) angeordneten Lichtwellenleiters (8) selbst die Lochblende (23) darstellt.

# Fig. 1

## Fig.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 145 563 (CENTRE NATIONAL DE LA RECHERCHE SCIENTIFIQUE) * Seite 7, Zeile 18 - Seite 14, Zeile 18 * * Figuren 1, 2 * | 1,3 | A 61 B 3/12 |
| Y,A | US-A-4 702 576 (P.C. MAGNANTE) * Spalte 4, Zeile 46 - Spalte 5, Zeile 67 * * Spalte 8, Zeilen 3 - 61; Figuren 3, 5-10 * | 1,3,4,5 | |
| D,A | US-A-3 698 099 (T.T. MATSURA) * Spalte 1, Zeile 61 - Spalte 3, Zeile 34 * | 1,2 | |
| A | MEDICAL AND BIOLOGICAL ENGINEERING AND COMPUTING. vol. 19, no. 1, Januar 1981, STEVENAGE GB Seiten 124 - 126; H.P.M. ESSINK et al.: "A quasi Maxwellian-view stimulator and ophthalmoscope with fibre optics" * das ganze Dokument * | 1,5 | |

| | RECHERCHIERTE SACHGEBIETE (Int. Cl.5) |
|---|---|
| | A 61 B |

Der vorliegende Recherchenbericht wurde für alle Patentansprüche erstellt

| Recherchenort | Abschlußdatum der Recherche | Prüfer |
|---|---|---|
| Den Haag | 25 Januar 91 | RIEB K.D. |